Europäisches Patentamt

European Patent Office

Office européen des brevets

(11) Veröffentlichungsnummer: **0 157 247**
**A2**

(19)

(12) # EUROPÄISCHE PATENTANMELDUNG

(21) Anmeldenummer: 85102928.0

(51) Int. Cl.⁴: **G 01 N 27/74**

(22) Anmeldetag: **14.03.85**

(30) Priorität: **04.04.84 CH 1700/84**

(43) Veröffentlichungstag der Anmeldung: **09.10.85**
**Patentblatt 85/41**

(84) Benannte Vertragsstaaten: **AT BE CH DE FR GB IT LI NL SE**

(71) Anmelder: **CERBERUS AG, Alte Landstrasse 411, CH-8708 Männedorf (CH)**

(72) Erfinder: **Forster, Martin, Sonnenbergstrasse 16, CH-8645 Jona (CH)**
Erfinder: **Strässler, Sigfrid, Segeihalde 40, CH-5405 Baden-Dättwil (CH)**
Erfinder: **Pfister, Gustav, Forbüelstrasse 17, CH-8707 Uetikon a/See (CH)**
Erfinder: **Eberle, Jakob, Ramensteinweg 10, CH-8708 Männnedorf (CH)**

(74) Vertreter: **Tiemann, Ulrich, Dr.-Ing., c/o Cerberus AG Patentabteilung Alte Landstrasse 411, CH-8708 Männedorf (CH)**

(54) **Verfahren und Vorrichtung zum Nachweis von reduzierenden Gasen in einem Gasgemisch.**

(57) Reduzierende Gase können in einem zu untersuchenden Gasgemisch, insbesondere in Luft, mit erhöhter Empfindlichkeit und Genauigkeit mittels eines Gasdetektors (100) nachgewiesen werden, bei welchem ein Gassensor (11) in einer Meßkammer (7) angeordnet ist und das zu untersuchende Gasgemisch periodisch gegen reines Referenzgas aus einer Referenzkammer (5) ausgetauscht wird. Während einer Vielzahl von Gasaustauschperioden (27) wird das auf der veränderten elektrischen Leitfähigkeit des Gassensors (11) beruhende Ausgangssignal des Gasdetektors (100) gemessen. Dieses Ausgangssignal setzt sich aus einem Gleichspannungssignal und einem bei der Frequenz der Gasaustauschperiode liegenden Wechselspannungssignal zusammen. Unter Zuhilfenahme von Kenngrössen des Gassensors (11) kann aus der Amplitude (66) des Wechselspannungssignals und dem Gleichspannungssignal (65) die Anwesenheit eines bestimmten reduzierenden Gases, vorzugsweise dessen genaue Konzentration, in Gegenwart von anderen reduzierenden Gasen bestimmt werden.

Verfahren und Vorrichtung zum Nachweis von
reduzierenden Gasen in einem Gasgemisch

Die Erfindung betrifft ein Verfahren zum Nachweis von reduzierenden Gasen in einem Gasgemisch gemäss Oberbegriff des Patentanspruchs l, sowie eine Vorrichtung zur Durchführung des Verfahrens.

In der DE-PS 23 13 413 ist ein Verfahren zum Nachweis des Kohlenmonoxid- und/oder des Methan-Gehalts eines Gasgemischs im
Untertagbetrieb beschrieben. Der Gehalt an den genannten Gasen
wird mit Hilfe einer Messung des elektrischen Widerstandes
eines Metalloxidhalbleiters, der die zu messende Gaskomponente
adsorbiert und bei höheren Temperaturen beschleunigt desorbiert,
bestimmt, indem vor jeder Messung die Temperatur des Metalloxidhalbleiters von einem unteren auf einen oberen Grenzwert geändert und die Messung bei dem unteren Grenzwert durchgeführt
wird. Dabei wird die Aenderung des elektrischen Widerstandes
über ein Zeitintervall gemessen und aus dem absoluten Messwert
und/oder dem zeitlichen Verlauf des Messignals die Konzentration
von Kohlenmonoxid und/oder Methan bestimmt.

Das in der genannten DE-PS beschriebene, auf die Belange des
Bergbaues abgestimmte Verfahren weist den Vorteil auf, dass
durch das periodische Aufheizen des Metalloxidhalbleiters eine
gute Langzeitstabilität erreicht wird. Beim Nachweis von Kohlenmonoxid ist zur Kompensation der Querempfindlichkeit gegen $CH_4$-
Komponeten einezweite, einen ständig geheizten Metalloxidhalbleiter aufweisende Messkammer erforderlich.

Die Regeneration des Metalloxidhalbleiters zwischen den einzelnen Messphasen dauert etwa 1 bis 5 Minuten, anschliessend ist die Abkühlung des Metalloxidhalbleiters auf Zimmertemperatur erforderlich, ehe der eigentliche Messvorgang beginnen kann. Das Verfahren ist daher für Alarmanlagen, bei denen es auf eine rasche Erkennung einer gefährlichen Konzentration an brennbaren Gasen ankommt, nicht geeignet. Da nur der absolute Messwert, bzw. der zeitliche Verlauf des Messsignals zur Bestimmung der Gaskonzentration benutzt wird, ist das Verfahren ausserdem nicht sehr empfindlich.

Aus der DE-OS 2 832 828 ist ein Kohlenmonoxiddetektor bekannt, der zur Verringerung des Einflusses anderer in einer Gasprobe zugleich vorhandener Gase zwei Kohlenmonoxiddetektoren unterschiedlicher Empfindlichkeit aufweist. Die unterschiedliche Kohlenmonoxidempfindlichkeit wird durch eine unterschiedliche Struktur der in den Kohlenmonoxiddetektoren enthaltenen Metalloxidhalbleiter oder durch eine unterschiedliche Temperatur der Detektorelemente erreicht. Für andere Gase ist dieses Prinzip nicht wirksam.

In der japanischen, offengelegten Patentanmeldung Sho 49-11997 ist ein Gas- und Rauchdetektor beschrieben, der zur Unterdrückung der Empfindlichkeit gegen langsame Aenderungen der Eigenschaften der Luft, wie z.B. Temperatur und Feuchtigkeit, zwei unterschiedlich der Luft zugängliche Messkammern mit Metalloxidhalbleitersensoren aufweist. Die Differenz der Messignale der beiden Sensoren wird für die Auswertung ausgenutzt. Wegen der leichten Diffundierbarkeit, insbesondere von niedermolekularen Gasen, ist die Differenz der beiden Messignale bald wieder Null, so dass der Detektor eher für den Nachweis von Rauch geeignet sein dürfte.

In der GB-PS 1 427 515 ist ein Detektor zum Nachweis von explosiven Gasen beschrieben, bei dem der Gasnachweis mit einem Pellistor erfolgt. Der Pellistor besteht aus einem auf Aluminiumoxid niedergeschlagenen Palladium/Thoriumdioxid-Gemisch und einem im Inneren befindlichen elektrischen Widerstandsthermometer. Die Messung erfolgt durch Temperaturvergleich zwischen dem Pellistor und einem zweiten Pellistor ohne Katalysator, bei dem durch Behandlung mit Kaliumhydroxid sichergestellt ist, dass jegliche Katalysatorwirkung unterbunden ist. Der Detektor arbeitet sehr träge.

In der DE-AS 10 17 384 ist ein Verfahren zum Nachweis von brennbaren Gasen beschrieben, bei dem der Nachweis durch katalytische Verbrennung der nachzuweisenden Gase an einem Platinglühfaden, dessen Widerstandsänderung als Messgrösse benutzt wird, erfolgt. Dabei dient der Platinglühfaden abwechselnd als Verbrennungsmasse und als Vergleichsmasse, indem über den Platinglühfaden abwechselnd das zu untersuchende Gasgemisch und ein Referenzgas mit keinem oder einem geringeren Gehalt an brennbaren Bestandteilen geleitet wird. Die Temperatur des Platinglühfadens schwankt zeitlich zwischen einem Höchstwert (Verbrennungsperiode) und einem Kleinstwert (keine brennbaren Bestandteile enthaltendes Referenzgas). Die Auswertung dieser Temperaturschwankungen ergibt ein Wechselspannungssignal, dessen Frequenz von der Austauschperiode des Gases und dessen Amplitude von dem Gehalt an brennbaren Bestandteilen abhängt. Letztere wird dann zur Bestimmung des Gehalts an brennbaren Gasen herangezogen.

Durch das in der DE-AS beschriebene Verfahren kann zwar ein Nachteil der mit katalytischer Verbrennung arbeitenden Gasdetektoren vermieden werden, nämlich die von physikalischen Veränderungen des Katalysators herrührende Wanderung des Nullpunkts. Andere Nachteile dieser Detektoren, wie beispielsweise ihre Empfindlichkeit gegenüber Vergiftungen des Katalysators durch Störgase oder ihre relative Unempfindlichkeit, konnten durch das in der DE-AS 10 17 384 vorgeschlagene Verfahren nicht behoben werden. Insbesondere ein Nachteil der nach dem Prinzip der katalytischen Verbrennung arbeitenden Gassensoren konnte nicht behoben werden: Die Temperatur des Platinglühfadens wird durch die Verbrennungswärme der nach zuweisenden brennbaren Gase bestimmt und der elektrische Widerstand des Platindrahtes ist von der Temperatur abhängig. Die Temperatur des Platinglühfadens hängt aber noch von einer Reihe anderer Bedingungen ab, so dass die erhaltenen Resultate nicht eindeutig interpretierbar sind.

Da die erhaltenen elektrischen Signale von der Temperatur des Platinglühfadens abhängig sind, sind sie auch von der durch die Geometrie der Messkammer bedingten Wärmeleitfähigkeit der Mess-, bzw. Brennkammer abhängig. Bereits geringfügige, zufällige Abweichungen in der Grösse und Dicke des Platinglühfadens und seiner Lage zum umgebenden Raum und dessen Wänden, ergeben Unterschiede in der Signalgrösse bis zum Faktor zehn. In gleicher Weise können Störungen durch unterschiedliche Strömungsverhältnisse und zufällige Ablagerungen in der Nähe des Platinglühfadens, die eine Aenderung der Wärmeleitfähigkeit mit sich bringen, nicht eliminiert werden.

Ueberraschender Weise wurde nunmehr gefunden, dass es bei einem Verfahren der eingangs definierten Art möglich ist, die Nachteile der mit katalytischer Verbrennung an Katalysatoren arbeitenden Verfahren dadurch zu beseitigen, dass man Gassensoren verwendet, deren elektrische Leitfähigkeit direkt von der Konzentration an reduzierenden Gasen in den zu untersuchenden Gasgemischen abhängt, dass man das sich aus der periodisch ändernden Leitfähigkeit ergebende Signal, das

0157247

sich aus einem konstanten Anteil und einem mit der Frequenz
des Luftbewegungsgenerators modulierten, sein Vorzeichen
wechselnden Anteil zusammensetzt, in einer Auswerteschaltung
in ein Gleichspannungssignal und ein bei der Frequenz des
Luftbewegugnsgenerators liegendes Wechselspannungssignal aufteilt und dass man die Amplitude des Wechselspannungssignals
und insbesondere das Verhältnis ihrer Grösse zur Grösse des
Gleichspannungssignals zum Nachweis von reduzierenden Gasen
auswertet.

Unerwarteter Weise ist dieses Verfahren auch den üblichen
mit Halbleitern arbeitenden Verfahren weit überlegen, was
Empfindlichkeit, Selektivität und Querempfindlichkeit anbelangt. Vor allem weist es den enormen Vorteil auf, dass aus
der Beziehung zwischen Wechselspannungssignal und Gleichspannungssignal auf die Art des reduzierenden Gases geschlossen werden kann.

Die Aufgabe der vorliegenden Erfindung besteht darin, die
vorstehend genannten Nachteile der Verfahren des Standes der
Technik zu beseitigen und die Verfahren zum Nachweis von reduzierenden Gasen so zu verbessern, dass die Signale des
Gassensors weniger abhängig vom Wassergehalt der Luft und
der Vorgeschichte des Gassensors werden, dass veränderliche
Umweltbedingungen, wie Druck und Temperatur, die Signale weniger beeinflussen, dass die Empfindlichkeit der Gassensoren
erhöht und ihre Querempfindlichketi verringert wird.

Eine wietere Aufgabe der vorliegendenErfindung besteht darin,
zu bestimmen, welche reduzierenden Gäse das zu untersuchende
Gasgemisch enthält und welche Konzentration ein bestimmtes
reduzierendes Gas in Gegenwart von anderen reduzierenden
Gasen aufweist.

Dies wird erfindungsgemäss bei einem Verfahren der eingangs
definierten Art durch die im kennzeichnenden Teil des Patentanspruchs 1 angegebenen Merkmale erreicht. In den abhängigen
Patentansprüchen werden bevorzugte Ausführungsformen der
Erfindung und besondere Ausgestaltungen definiert.

Gemäss einer Ausführungsform des erfindungsgemässen Verfahrens wird das zu untersuchende Gasgemisch mittels eines Kolbens durch den mit einer Heizung beheizten Gassensor hindurch
oder über diesen hinweg in der Weise gesaugt und dann zurückgedrückt, dass praktisch das gesamte in der Messkammer befindliche zu untersuchende Gasgemisch mit der Oberfläche des
Gassensors in Berührung kommt, wodurch eine vollständige
Umsetzung der reduzierenden Gase an der Oberfläche des Gassensors erfolgt und beim Ausblasen der Messkammer durch zurückdrücken des Kolbens ein praktisch von reduzierenden Gasen
freies zu untersuchendes Gasgemisch den Gassensor passiert.

Als Gassensor kann im erfindungsgemässen Verfahren vorzugsweise ein Metalloxid-Halbleitergassensor verwendet werden.

Gemäss einer bevorzugten Ausführungsform wird das Volumen
der Teile, die das zu untersuchende Gasgemisch passiert, möglichst gering gehalten, damit das Totvolumen auf ein Minimum
reduziert wird.

Bei einer Anordnung zur Durchführung des erfindungsgemässen Verfahrens sind die Volumina von Messkammer und Referenzkammer so aufeinander abgestimmt, dass bei jedem Einsaugen frisches zu untersuchendes Gasgemisch in die Messkammer eingesaugt und bei jedem Ausblasen mindestens ein Teil des zu untersuchenden Gasgemisches durch die Einlassöffnung die Messkammer verlässt. Es kann auch eine von der Einlassöffnung getrennte Auslassöffnung vorgesehen sein.

Die Vorrichtung zum Einsaugen und Ausblasen des zu untersuchenden Gases besteht aus einem üblichen Luftbewegungsgenerator. Dieser Luftbewegungsgenerator bildet vorzugsweise eine Wand oder einen Wandteil der Referenzkammer, durch deren Bewegung das Volumen der Referenzkammer periodisch mit einer bestimmten Frequenz verändert wird. Diese Frequenz beträgt vorzugsweise zwischen 0,01 und 50 Hz, insbesondere zwischen 0,1 und 10 Hz; besonders bevorzugt ist eine Frequenz von etwa 2 Hz.

Gemäss bevorzugten Ausführungsformen der Erfindung kann der Luftbewegungsgenerator z.B. als elektromagnetisch, elektrostatisch, piezoelektrisch oder thermomechanisch erregbare Membran in der Art eines Lautsprechers ausgebildet sein, eine Piezo-Folie vom Typ Polyvinylidendifluorid (PVDF) aufweisen, ein bimorphes piezoelektrisches oder bimetallisches Element enthalten oder eine durch Mikrolithographie erzeugte dünne Siliziumfolie aufweisen.

Gemäss einer besonders bevorzugten Ausführungsform dient ein Philips-Woofer AD 4060/W4 als Luftbewegungsgenerator.

Gemäss einer weiteren bevorzugten Ausführungsform wird die Referenzkammer so gestaltet, dass ihr Volumen grösser ist als das Volumen der Messkammer. Dadurch wird einerseits sichergestellt, dass der Inhalt der Messkammer bei jeder Einsaug/Ausblas-Periode durch frisches Gasgemisch ersetzt wird, und andererseits wird bei Ausbildung einer Referenzkammerwand als schwingende Membran mit relativ grosser Fläche schon bei geringer Schwingungsamplitude der Wand das Gas in der Messkammer weitgehend oder fast vollständig und mit geringem Energieaufwand ausgetauscht. Gemäss einer weiteren Ausführungsform wird vor der schwingenden Membran eine Schutzmembran angebracht, welche die sich bewegende Membran vor der Einwirkung erhöhter Temperatur schützt.

Gemäss einer weiteren bevorzugten Ausführungsform wird die Referenzkammer so gestaltet, dass ihr Volumen beim Ausblasen praktisch Null wird. Dies wird vorzugsweise dadurch erreicht, dass der Luftbewegungsgenerator als schwingende Membran ausgebildet wird, die sich beim Ausblasen dicht an eine, z.B. konusförmige, Auflage anlegt.

Gemäss weiteren Ausgestaltungen des in dem erfindungsgemässen Verfahren angewendeten Gasdetektors befindet sich in der Referenzkammer, vorzugsweise in der Oeffnung, bzw. den Oeffnungen zwischen Mess- und Referenzkammer ein zusätzliches Filter zur Adsorption von Gasen. Zusätzlich oder anstelle dieses Filters kann ein entsprechendes Filter vor der Einlassöffnung angeordnet werden. Diese Filter sind durch Ausheizen in gewissen Zeitabständen regenerierbar.

0157247

Gemäss einer weiteren Ausführungsform des im erfindungsgemässen Verfahren angewendeten Gasdetektors kann sich in der Referenzkammer ein zweiter Sensor, ein Referenzsensor, befinden, der z.B. nur Wasserdampf misst.

Die Auswerteschaltung erfasst die elektrische Leitfähigkeit
des Gassensors und erzeugt ein Spannungssignal, das über eine
mathematische Funktion mit σ verknüpft ist. Die Form dieser
mathematischen Funktion kann entweder linear oder nichtlinear, z.B. logarithmisch, in σ sein und ist durch die gewählte
elektronische Schaltung in der Auswerteschaltung bestimmt.
Dieses Spannungssignal besteht in der vorliegenden Erfindung
aus einem Gleichspannungssignal (DC-Signal) und einem diesem
überlagerten, bei der Frequenz des Luftbewegungsgenerators
liegenden Wechselspannungssignal (AC-Signal). Die Auswerteschaltung trennt diese beiden Signale und bestimmt die Amplitude des bei der Frequenz des Luftgenerators liegenden Wechselspannungssignals. Diese AC-Amplitude ist nun ein Mass für
die Konzentrationen der im zu untersuchenden Gasgemisch enthaltenen reduzierenden Gase, und durch einen Vergleich mit
dem Gleichspannungssignal bei gegebener AC-Amplitude, was
ebenfalls durch geeignete elektronische Schaltelemente in
der Auswerteschaltung erreicht wird, kann sogar auf die Art
der reduzierenden Gase geschlossen werden und auf die Konzentration eines bestimmten reduzierenden Gases in Anwesenheit
anderer reduzierender Gase.

Bei einer weiteren bevorzugten Ausführungsform des im erfindungsgemässen Verfahren angewendeten Gasdetektors können auch
die Messkammer und die Referenzkammer ohne Einschnürung
direkt ineinander übergehen, d.h. der Gassensor befindet sich
in einer Kammer, die sowohl Messkammer als auch Referenzkammer ist und von der mindestens eine Wand oder ein Teil davon
als Luftbewegungsgenerator ausgebildet ist.

Die Erfindung, sowie zweckmässige Weiterbildungen derselben werden anhand der in den Figuren dargestellten Ausführungsbeispielen näher erläutert. Es zeigen:

| | |
|---|---|
| Fig. 1 | einen vereinfachten Querschnitt durch einen erfindungsgemässen Gasdetektor, |
| Fig. 2a | ein Schaubild der elektrischen Leitfähigkeit σ (angegeben in $\Omega^{-1}$) des Gassensors in Abhängigkeit von der Zeit t, |
| Fig. 2b | die Konzentration des reduzierenden Gases in der Messkammer, |
| Fig. 2c | die Flussrichtung durch die Messkammer, bestimmt an der Einlassöffnung, |
| Fig. 3 | eine graphische Darstellung des Spannungssignals, das in der Auswerteschaltung erzeugt wird, als Funktion der Zeit, |
| Fig. 4 | eine graphische Darstellung der AC-Amplitude des Wechselspannungssignals als Funktion der Konzentration an reduzierendem Gas, |
| Fig. 5 | den Zusammenhang zwischen DC-Wert und AC-Amplitude, |
| Fig. 6 bis 12 und Fig. 15 | Ausgestaltungen von Gasdetektoren, die in dem erfindungsgemässen Verfahren angewendet werden können, |
| Fig. 13 und 14 | elektronische Schaltungen zur Auswertung des DC- und AC-Signals. |

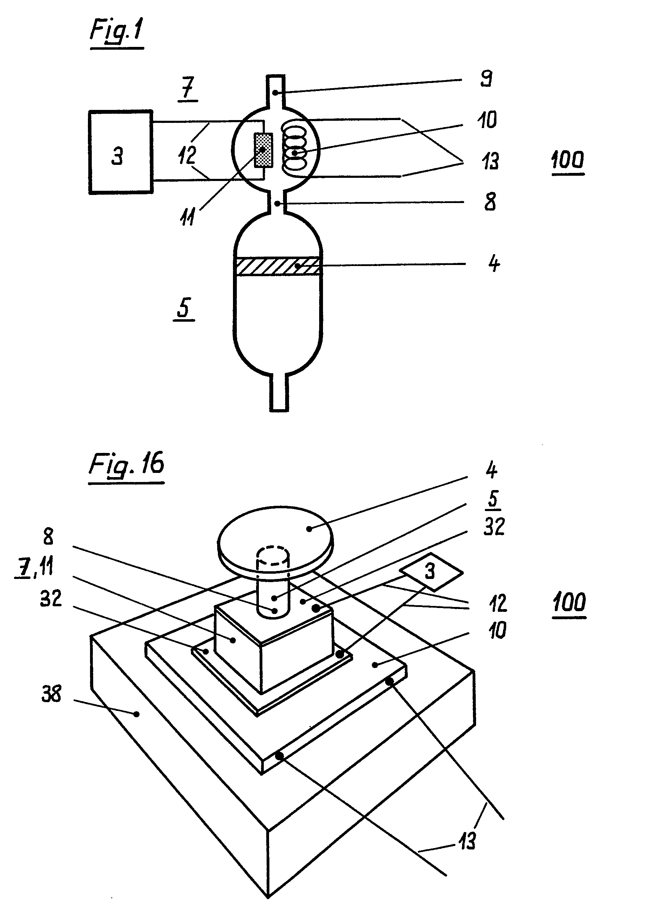

Das in Fig. 1 dargestellte Schema eines Gasdetektors 100 besteht im wesentlichen aus einer Messkammer 7 und einer Referenzkammer 5. In der Messkammer 7 befindet sich der Gassensor 11 mit der Heizung 10. Der Heizung 10 wird durch die elektrischen Leitungen 13 von einer nicht dargestellten Energiequelle elektrische Energie zugeführt. Die Heizung 10 kann den Gassensor 11 umgeben oder sie kann in das Material des Gassensors 11 eingelassen sein oder auf nur einer Seite oder auf zwei oder mehreren Seiten desselben angebracht sein. Der Gassensor 11 ist über die elektrischen Leitungen 12 mit einer Auswerteschaltung 3 verbunden. Handelt es sich beim Gassensor 11 um einen Pellistor, so sind die elektrischen Leitungen 12 und 13 identisch. Ueber die Einlassöffnung 9 wird der Messkammer 7 das zu untersuchende Gasgemisch zugeführt. Ueber die Verbindungsöffnung 8 ist die Messkammer 7 mit der Referenzkammer 5 verbunden, in der ein Luftbewegungsgenerator 4 angeordnet ist.

Im folgenden wird die Arbeitsweise eines in Fig. 1 schematisch dargestellten Gasdetektors 100 anhand der Fig. 2a bis 2c beschrieben und mit der Arbeitsweise eines bisher üblichen Gassensors verglichen (vgl. Fig. 4). Für beide Fälle wird als Gassensor 11 ein Metalloxidhalbleiter-Gassensor verwendet.

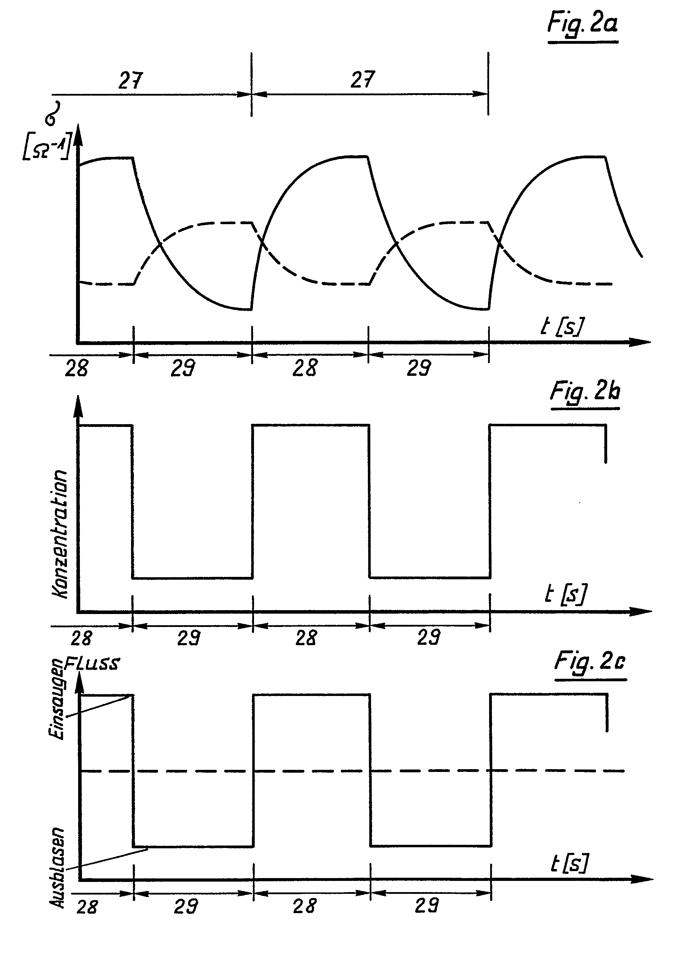

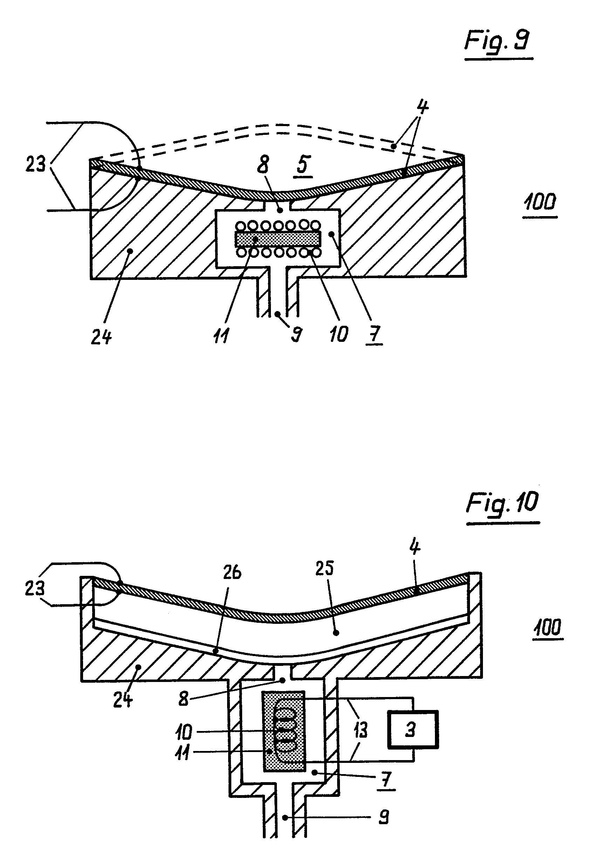

Der Gassensor 11 wird mittels der Heizung 10 auf eine vorbestimmte Temperatur, im vorliegenden Fall 350 °C, erhitzt. Ueber die elektrischen Leitungen 12 wird mittels einer Auswerteschaltung 3 die elektrische Leitfähigkeit $\sigma$ bestimmt. In der Fig. 2a ist die elektrische Leitfähigkeit $\sigma$ in $\Omega^{-1}$ gegen die Zeit t in Sek. (s) mit einer ausgezogenen Linie aufgezeichnet. Die gestrichelte Linie in Fig. 2a bezieht sich auf einen Gasdetektor 100, der als Gassensor 11 einen Pellistor enthält. Dieser Fall ist in der Beschreibung zur Fig. 10 genauer ausgeführt. In Fig. 2b ist die Konzentration an reduzierenden Gasen in der Messkammer und in Fig. 2c die Flussrichtung in der Messkammer an der Oeffnung 9 aufgezeichnet. Auf der Abszissenachse ist jeweils die Zeit für die Einsaugphase 28 und die Ausblasphase 29, die zusammen eine Gasaustauschperiode 27 bilden, angegeben.

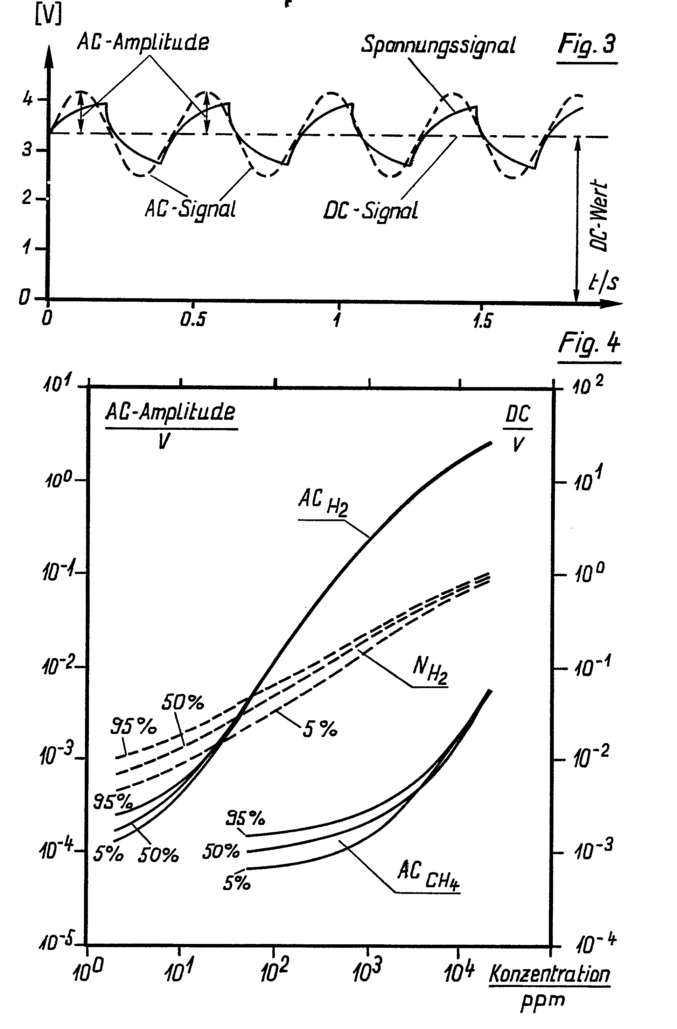

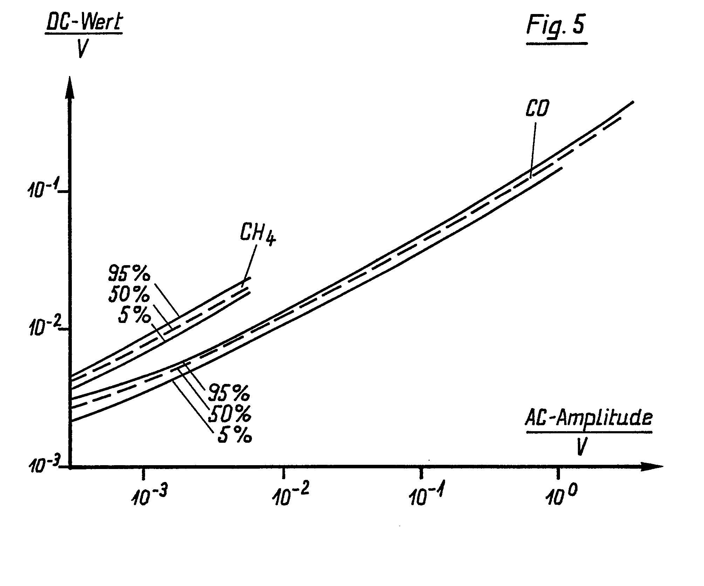

In Fig. 3 ist gegen die Zeit t in Sek. das elektrische Spannungssignal in Volt aufgezeichnet, das in der Auswerteschaltung 3 entsprechend der elektrischen Leitfähigkeit σ des Gassensors 11 erzeugt wird. Dieses Spannungssignal ist in den Messungen zu den Fig. 3 und 4 direkt proportional zur elektrischen Leitfähigkeit σ des Gassensors 11. Entsprechend der elektrischen Leitfähigkeit σ des Gassensors 11 besteht das in der Auswerteschaltung 3 erzeugte Spannungssignal aus einem Gleichspannungsanteil (DC-Signal), der in Fig. 3 strichpunktiert eingezeichnet ist, und einem diesen überlagerten, mit der Frequenz des Luftbewegungsgenerators 4 modulierten Wechselspannungsanteil (AC-Signal), der in Fig. 3 gestrichelt eingezeichnet ist. Dieser Wechselspannungsanteil besitzt eine AC-Amplitude, die direkt von der Konzentration der im zu untersuchenden Gasgemisch enthaltenen reduzierenden Gase abhängt. Das Verhältnis von AC-Amplitude zu DC-Wert ist für jedes gegebene reduzierende Gas bei gegebener AC-Amplitude charakteristisch. Das Verhältnis AC-Amplitude:DC-Wert ist von der Länge der Periode 27, d.h. von der Frequenz des Wechselspannungssignals und damit von der Frequenz des Luftbewegungsgenerators 4 abhängig. Je tiefer die Frequenz des Luftbewegungsgenerators 4 ist, desto grösser ist das Verhältnis AC-Amplitude:DC-Wert. Hält man jedoch die Frequenz konstant, d.h. lässt man die Länge der Periode 27 unverändert, so ist das Verhältnis AC-Amplitude:DC-Wert bei gegebener AC-Amplitude nur noch gasabhängig und kann somit zur Bestimmung der Art des nachzuweisenden reduzierenden Gases benutzt werden (siehe Fig. 5).

Trägt man die für Wasserstoff erhaltenen Werte der AC-Amplitude in Abhängigkeit von der Gaskonzentration in doppeltlogarithmischem Massstab auf, so erhält man die in Fig. 4 dargestellten Linien $AC_{H2}$. Die drei Linien repräsentieren
Messungen, die mit 5 %, 50 % und 95 % relativer Luftfeuchte
bei 27 °C durchgeführt worden sind. Die für Kohlenmonoxid
erhaltenen Kurven sind mit den Kurven $AC_{H2}$ für Wasserstoff
praktisch identisch, insbesondere weisen beide Kurvenscharen
eine Steigung auf, die einem Exponenten 3/2 entspricht, siehe
Gleichung (1).

Ist das nachzuweisende Gas Methan, so erhält man die Kurven
$AC_{CH4}$, die wieder mit relativen Luftfeuchten von 5 %, 50 %
und 95 % bei 27 °C gemessen worden sind. Die AC-Amplituden
sind hier bei gleicher Gaskonzentration erheblich geringer.
Die Kurven weisen aber für hohe Methankonzentrationen ebenfalls den Exponenten 3/2 auf.

In Fig. 4 stellen die gestrichelt gezeichneten Linien $N_{H2}$
das Ausgangssignal eines normalen Gassensors für Wasserstoff
dar. Das Ausgangssignal (rechts angebrachte Skala) ist in
doppeltlogarithmischem Massstab gegen die Konzentration aufgetragen. Es ist deutlich der flachere Verlauf der Kurven
$N_{H2}$ zu erkennen, der einem Exponenten 1/2 entspricht.

Bei einem linearen Zusammenhang zwischen $\sigma$ und dem Wechselspannungssignal, das durch die Auswerteschaltung 3 erzeugt
wird, ergibt sich für den im erfindungsgemässen Verfahren
verwendeten Gasdetektor 100, dass sich die AC-Amplitude über
weite Konzentrationsbereiche proportional zur (Gaskonzentration)$^{3/2}$ ändert. Dies kann in der folgenden Formel zum Ausdruck gebracht werden:

$$\text{AC-Amplitude}_{Gas} = \alpha \cdot [Gas]^{3/2} \qquad (1)$$

(worin α ein Proportionalitätsfaktor ist), d.h. die AC-Amplitude ändert sich mit der dritten Potenz der Quadratwurzel aus der Gaskonzentration. Der Exponent 3/2 ist unabhängig von der Gasart, tritt aber bei schwieriger oxidierbaren Gasen, wie Methan, erst bei höheren Konzentrationen auf. Der Exponent 3/2 für eine konzentrationsabhängige Grösse ist für Gasdetektoren neu und stellt einen erheblichen Fortschritt dar für die Konstruktion hochempfindlicher und präziser Gasdetektoren.

Weiterhin zeigt Fig. 4, dass die AC-Amplituden von $H_2$ praktisch im gesamten Konzentrationsbereich und für $CH_4$ im höheren, hauptsächlich interessierenden Konzentrationsbereich unabhängig sind von der relativen Luftfeuchte. Dies stellt einen weiteren, grossen Fortschritt dar für die Konstruktion von Gasdetektoren, deren Signale durch variierende Luftfeuchtigkeit nicht beeinflusst werden. Diese vorzügliche Eigenschaft der erfindungsgemässen Gasdetektoren rührt davon her, dass die Gasgemische, die sich abwechselnd während des Einsaugens 28 und des Ausblasens 29 in der Messkammer 7 befinden, praktisch denselben Wasserdampfgehalt aufweisen. Durch den Gassensor 11 werden ja nur die reduzierenden Gase aus dem angesaugten Gasgemisch ganz oder mindestens teilweise entfernt, nicht aber der im Gasgemisch enthaltene Wasserdampf. Da die AC-Amplitude aber nur durch Aenderungen der Gaszusammensetzung zwischen der Einsaugphase 28 und der Ausblasphase 29 beeinflusst wird, hat der Wasserdampfgehalt des zu untersuchenden Gasgemisches nur einen sehr kleinen Einfluss auf die AC-Amplitude.

Entsprechend der Aufteilung der elektrischen Leitfähigkeit σ in einen konstanten Anteil und einen diesem überlagerten, mit der Frequenz des Luftbewegungsgenerators modulierten Anteil, verändert sich bei einer Aenderung der Konzentration an reduzierenden Gasen auch der konstante Anteil von $\sigma$ . Sobald sich die Konzentration an reduzierenden Gasen in dem zu untersuchenden Gasgemisch ändert, ändert sich deshalb neben der AC-Amplitude auch der DC-Wert.

Fig. 5 zeigt den Zusammenhang zwischen dem DC-Wert und der AC-Amplitude eines erfindungsgemässen Gasdetektors 100 für zwei verschiedene Gase, nämlich Methan und Kohlenmonoxid, in Luft, wobei sich die Messungen auf Gasgemische mit 5 % (untere Kurve) bis 95 % (obere Kurve) relative Luftfeuchte beziehen. Die Messungen sind bei 27 °C durchgeführt worden.

Fig. 5 zeigt eindeutig, dass bei gleich grosser AC-Amplitude für Methan und Kohlenmonoxid der DC-Wert für Methan signifikant grösser ist als für Kohlenmonoxid. Dies gilt für alle Luftfeuchtigkeiten, d.h. durch den Vergleich zwischen DC-Wert und AC-Amplitude kann auch unter ungünstigen klimatischen Verhältnissen eindeutig entschieden werden, ob die Signale des Gassensors von z.B. Kohlenmonoxid oder von Methan herrühren. Eine solche eindeutige Unterscheidung, die mit einem einzigen Sensor und rein rechnerisch erfolgen kann, ist bis jetzt noch nicht bekannt und stellt einen grossen Fortschritt dar zur selektiven Detektion von spezifischen Gasen.

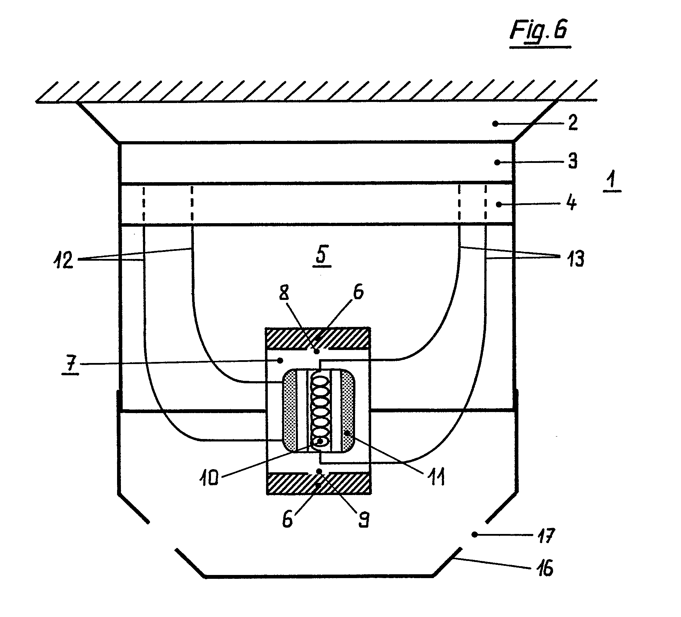

Die in Fig. 6 dargestellte, speziell zum Nachweis von reduzierenden Gasen geeignete Anordnung zur Gasdetektion besteht aus einem Detektoreinsatz 1, der in einen Sockel 2, z.B. einen üblichen Gasmeldersockel, einsetzbar ist. Der Detektoreinsatz 1 enthält eine elektrische Steuer- und Auswerteschaltung 3, oder zumindest einen Teil derselben, wobei der Rest der Schaltung in einer über Leitungen mit dem Sockel 2 verbundenen, nicht dargestellten Signalzentrale untergebracht sein kann. Von der Auswerteschaltung 3 wird ein Luftbewegungsgenerator **4** angesteuert, der eine Wand der anschliessenden Referenzkammer **5** oder einen Teil derselben bildet und deren Volumen sich periodisch mit einer bestimmten Frequenz, vorzugsweise zwischen 0,1 und 10 Hz, z.B. 2 Hz, ändert. Der Luftbewegungsgenerator **4** kann z.B. als elektromagnetisch, elektrostatisch, piezoelektrisch oder thermomechanisch erregbare Membran in der Art eines Lautsprechers ausgeführt sein oder eine Piezo-Folie vom Typ PVDF aufweisen oder ein bimorphes, piezoelektrisches oder bimetallisches Element enthalten. In miniaturisierten Gasdetektoren nach dem erfindungsgemässen Verfahren kann der Luftbewegungsgenerator **4** auch eine durch Mikrolithographie erzeugte Siliziummembran sein. In einem praktisch ausgeführten Beispiel erwies sich als Luftbewegungsgenerator ein Philips-Woover AD 4060/W4 als geeignet.

Die Referenzkammer **5** ist ausser der Verbindungsöffnung **8** zur Messkammer **7** vollständig gegenüber der Aussenatmosphäre abgeschlossen. Sie dient als Reservoir für das von reduzierenden Gasen mindestens teilweise, vorzugsweise vollständig, befreite Gasgemisch.

Die Referenzkammer **5** steht mit der Messkammer **7** über eine Verbindungsöffnung **8** geringen Querschnitts (ca. 0,03 cm$^2$) in Verbindung. In der Messkammer **7** befindet sich der Gassensor **11**, im vorliegenden Fall ein normaler Gassensor TGS-812 (Taguchi/Figaro), der durch eine Heizung **10**, die über elektrische Leitungen **13** mit elektrischer Energie versorgt wird, auf eine Temperatur im Bereich 80 bis 650 °C aufgeheizt werden kann. Als beste Arbeitstemperatur hat sich die Temperatur von 350 °C erwiesen. Der Gassensor **11** ist über elektrische Leitungen **12** mit der Auswerteschaltung **3** verbunden.

Die Messkammer 7 weist ein Volumen von ca. 0,10 $cm^3$ auf, während die Referenzkammer 5 ein Volumen von ca. 100 $cm^3$ aufweist, so dass das Volumenverhältnis mehr als 1 zu 1000 beträgt. Das Verhältnis der Summe der Volumina von Referenzkammer 5 und Messkammer 7 zur Summe der Volumina von Gassensor 11 und Heizung 10 beträgt in diesem Beispiel $2 \times 10^4:1$. In einer zur Fig. 6 analogen erfindungsgemässen Anordnung zur Gasdetektion wurde anstelle eines kommerziellen TGS-812-Metalloxidgassensors mit einem Volumen von ca. 5 $mm^3$ für Sensor und Heizung ein Miniaturgassensor eingesetzt, der ein Volumen für Sensor und Heizung von nur 0,05 $mm^3$ aufwies. Obwohl das Verhältnis der Summe der Volumina von Referenzkammer 5 und Messkammer 7 zur Summe der Volumina von Gassensor 11 und Heizung 10 in diesem Fall sogar $2 \times 10^7:1$ betrug, arbeitete diese erfindungsgemässe Anordnung zur Gasdetektion ebenfalls äusserst zufriedenstellend. Bei einer Schwingung des Luftbewegungsgenerators 4 wird ein der Schwingungsamplitude entsprechendes Volumen des zu untersuchenden Gasgemisches durch die Einlassöffnung 9 und durch die Messkammer 7 hindurch am Gassensor 11 vorbei in die Referenzkammer 5 gesaugt. Bei dieser Phase des Einsaugens 28 (vgl. Fig. 2) wird das zu untersuchende Gasgemisch mindestens teilweise von reduzierenden Gasen befreit. In umgekehrter Richtung wird durch eine Schwingung des Luftbewegungsgenerators 4 ein mindestens teilweise von reduzierenden Gasen befreites Gasgemisch (Referenzgas) durch die Verbindungsöffnung 8 in die Messkammer 7 hineingedrückt und verlässt diese durch die Einlassöffnung 9 (Phase des Ausblasens 29).

In der Referenzkammer 5 kann vor der Verbindungsöffnung 8 ein Filter 6 zur Adsorption von Restmengen von reduzierenden Gasen oder anderen störenden Gasen angeordnet sein. Das Filter 6 wird durch Ausheizen in gewissen Zeitabständen regeneriert. Ein Filter zur Adsorption störender Gase kann auch vor der Einlassöffnung 9 angeordnet sein. Auf den Detektoreinsatz 1 kann ein Gehäuse 16 aufgesteckt werden, das Oeffnungen 17 zum Eintritt des zu untersuchenden Gases, in diesem Fall der Aussenatmosphäre, aufweist. Das Gehäuse 16 kann durch seine geometrische Gestaltung und durch die Anordnung der Oeffnungen 17 verschiedenen Umweltbedingungen angepasst werden. Die Einlassöffnung 9 der Messkammer 7 kann auch direkt über ein nicht dargestelltes Zuleitungsrohr mit dem zu untersuchenden Gas beschickt werden. Da das erfindungsgemässe Verfahren weitgehend windunabhängig ist, ist ein Gehäuse 16 nicht unbedingt erforderlich.

Zur Erhöhung der Windunabhängigkeit ist in einem weiteren Ausführungsbeispiel die Oeffnung 9 als eine ca. 1 cm lange Kapillare mit einem Innendurchmesser von ca. 0,3 mm ausgebildet. Damit wird die Gasströmung am Sensor nur noch durch die Bewegung des Luftbewegungsgenerators bestimmt, die Messungen der Gaskonzentration werden somit völlig unabhängig von der Strömungsgeschwindigkeit des zu untersuchenden Luft-Gas-Gemisches.

Beim Betrieb der beschriebenen Anordnung wird periodisch mit einer bestimmten Frequenz, z.B. 2 Hz, Luft zwischen der Referenzkammer 5 und der Messkammer 7 und damit auch zwischen der Messkammer 7 und der Aussenatmosphäre hin und her bewegt. Dabei sollte zweckmässigerweise das gesamte bewegte Luftvolumen mindestens etwa dem Volumen der Messkammer 7 entsprechen. Damit wird das Messkammervolumen mindestens teilweise, im Idealfall praktisch vollständig, periodisch ausgetauscht, und es befindet sich abwechselnd Aussenluft (d.h. das zu untersuchende Gasgemisch) oder Luft aus der Referenzkammer 5 (d.h. Gasgemisch ohne reduzierende Gase) in der Messkammer 7

0157247

und damit im Bereich des Gassensors 11. Das bewegte Volumen hängt von der speziellen geometrischen Ausbildung der beiden Kammern 5 und 7, der Oeffnung 8, des Filters 6 und der Bewegungsamplitude des Luftbewegungsgenerators 4 ab.

Solange sich in der Aussenluft keine reduzierenden Gase befinden, bleibt die elektrische Leitfähigkeit σ des Gassensors 11 unverändert, d.h. die Auswerteschaltung 3 erzeugt ein Gleichspannungssignal DC. Sobald aber die Aussenluft reduzierende Gase enthält, wechselt die Luft in der Messkammer 7 periodisch zwischen Luft mit geringerem Gehalt an reduzierenden Gasen aus der Referenzkammer 5 und Luft aus der Aussenatmosphäre, die reduzierende Gase enthält. Daher ändert sich auch die Leitfähigkeit σ des Gassensors 11 periodisch mit der Frequenz der Luftwechsel in der Messkammer. Die Auswerteschaltung 3 erzeugt nun gemäss der elektrischen Leitfähigkeit des Gassensors 11 ein Spannungssignal, das aus einem DC-Signal und einem diesen überlagerten AC-Signal besteht, wobei das letztere eine Frequenz aufweist, die mit der Frequenz des Luftbewegungsgenerators 4 übereinstimmt. Das AC-Signal kann von der Auswerteschaltung 3 auf einfache Weise vom DC-Signal getrennt werden. Die Auswerteschaltung 3 bestimmt nun die Amplitude des AC-Signals und ist so ausgelegt, dass sie bei Ueberschreiten eines bestimmten Wertes der AC-Amplitude ein Alarmsignal abgibt.

Durch eine Quotientenschaltung kann in der Auswerteschaltung 3 das Verhältnis des DC-Wertes zur AC-Amplitude gebildet werden.

Da das Verhältnis DC-Wert:AC-Amplitude bei gegebener AC-Amplitude bei konstanter Frequenz des Luftbewegungsgenerators 4 (und damit konstanter Frequenz des Wechselsignals) von der Art des reduzierenden Gases abhängt, kann die Auswerteschaltung 3 so ausgelegt werden, dass ein Alarmsignal in Abhängigkeit von der Grösse der AC-Amplitude nur abgegeben wird, wenn das Verhältnis DC-Wert:AC-Amplitude bei gegebener AC-Amplitude einen vorbestimmten Wert annimmt, d.h. wenn in dem nachzuweisenden Gasgemisch ein ausgewähltes und in gefährlicher Konzentration auftretendes Gas vorhanden ist.

Das erfindungsgemässe Verfahren ist nicht auf die Verwendung von Halbleitern als Gassensoren beschränkt. Es können anstelle von Halbleiter-Gassensoren auch optische Gassensoren verwendet werden, wie sie beispielsweise in den CH-PA 1 578/83, 1 579/83, 4 736/83, 4 737/83 und 4 738/83 beschrieben sind.

Sehr gute Resultate werden auch erzielt, wenn anstelle eines Halbleiters ein Pellistor verwendet wird, der nach dem Prinzip der katalytischen Verbrennung arbeitet.

Ferner ist das erfindungsgemässe Verfahren nicht darauf beschränkt, dass das nachzuweisende Gas durch Oxidation oder Reduktion, d.h. durch eine chemische Reaktion, aus dem zu untersuchenden Gasgemisch entfernt wird. Das nachzuweisende Gas kann auch beispielsweise durch Adsorption an einem Festkörper, z.B. auf einem Oszillator wie im Patent US 4 399 686, durch einen geeigneten Festkörperelektrolyten oder durch Herauspumpen durch eine Ionenpumpe (z.B. Sauerstoff) entfernt werden.

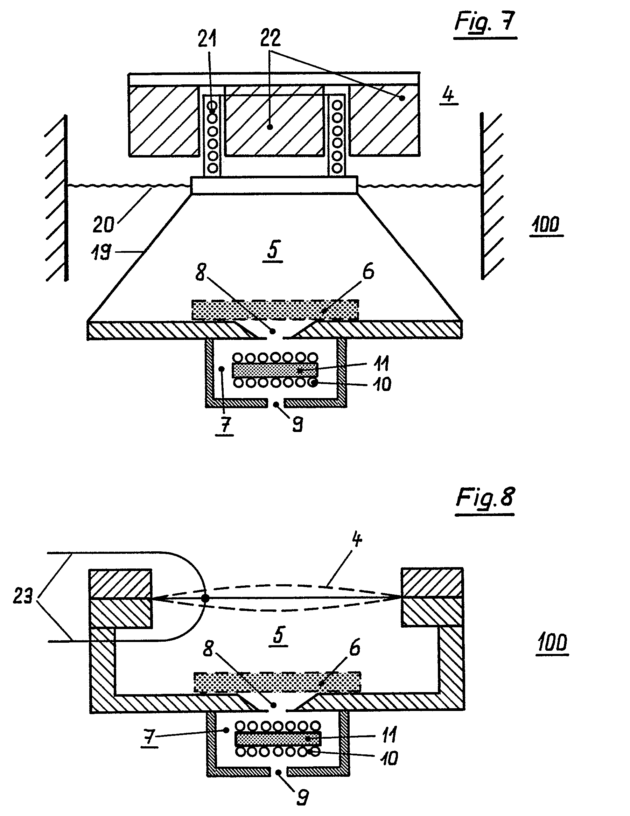

In Fig. 7 ist eine weitere Ausgestaltung eines in dem erfindungsgemässen Verfahren verwendeten Gasdetektors 100 dargestellt. Eine konisch ausgebildete Wand 19 der Referenzkammer
5 ist an einer Spule 21 befestigt, die von einer federnden
Halterung 20 getragen wird und in einen Ringspalt eines Permanent-Magneten 22 hineinragt. Bei Erregung der Spule 21
durch eine Wechselspannung gerät die konische Wand 19 wie
bei einem elektrodynamischen Lautsprecher in Schwingungen
und ändert dadurch das Volumen der Referenzkammer 5 periodisch. Bei dieser Schwingung wird die Luft periodisch aus
der Referenzkammer 5 durch das Filter 6 und die Oeffnung 8
in die Messkammer 7 hineingedrückt und wieder zurückgesogen.

In Fig. 8 ist eine weitere Ausgestaltung des Gasdetektors
100 dargestellt. Der Luftbewegungsgenerator ist als bimorphe
Piezo-Folie 4 ausgebildet. Je nach der an den beiden Seiten
mittels der Leitungen 23 angelegten Spannung erfährt diese
Piezo-Folie eine entsprechende Auslenkung. Wird nun an die
Leitungen 23 eine Wechselspannung angelegt, so gerät die
Piezo-Folie 4 in eine Schwingung, deren Frequenz der angelegten Wechselspannung entspricht. Das Volumen der Referenzkammer 5 schwankt mit der gleichen Frequenz.

In Fig. 9 ist eine Weiterentwicklung des in Fig. 8 beschriebenen Gasdetektors 100 dargestellt, wobei das Volumen der
Referenzkammer 5 im Endstadium der Ausblasphase 29
das Volumen Null hat, d.h. das Totvolumen verschwindet praktisch. Dies wird dadurch erreicht, dass in der Referenzkammer 5 eine konusförmige oder auch ebene Auflage 24 vorgesehen
ist, an die sich die Piezo-Folie 4 am Ende der Ausblasphase
29 dicht anlegt.      Dies ist in der Fig. 9 durch die ausgezogenen Linien dargestellt. Am Ende der Einsaugphase 28
nimmt    die Piezo-Folie 4 die punktiert dargestellte Lage
ein.

In Fig. 10 ist eine Weiterentwicklung des in Fig. 9 beschriebenen Gasdetektors 100 gezeigt. Die temperaturempfindliche,
den Luftbewegungsgenerator 4 bildende Piezo-Folie überträgt
ihre Bewegung via das Arbeitsvolumen 25 auf die Schutzmembran
26, die hier aus einer temperaturbeständigen, sehr dünnen
(1 - 10 μm) Metallmembran besteht.

Im Gegensatz zur Fig. 9, wo der Gassensor 11 ein Metalloxid-
Halbleitergassensor ist, wird hier ein bekannter Pellistor
als Gassensor 11 verwendet, dessen Heizung 10 aus einer
Drahtspirale besteht und für dieses Beispiel als Heizwicklung
bezeichnet wird, deren elektrischer Widerstand einen stark
positiven Tempeaturkoeffizienten aufweist. Die Zuleitungen
13 der Heizwicklung 10 sind in diesem Fall identisch mit den
Messleitungen 12 und enden in einer Auswerteschaltung 3.

Die gestrichelte Kurve in Fig. 2a zeigt die elektrische Leitfähigkeit σ dieser Heizwicklung 10 als Funktion der Zeit in
den Gasaustauschperioden 27. Während der Einsaugphase 28 verbrennen die im Gasgemisch enthaltenen  reduzierenden Gase
am Gassensor 11, der eine Temperatur von ca. 450 °C aufweist. Die bei dieser Oxidation frei werdende Verbrennungswärme heizt die Wicklung 10 zusätzlich auf, wodurch sich
deren Leitfähigkeit σ etwas erniedrigt. Während dieser Einsaugphase 28 wird das zu untersuchende Gasgemisch mindestens
teilweise von reduzierenden Gasen befreit. In umgekehrter
Richtung wird durch eine Bewegung der Schutzmembran 26 und
des Luftbewegungsgenerators 4 ein mindestens teilweise
von reduzierenden Gasen befreites Gasgemisch (Referenzgas)
durch die Verbindungsöffnung 8 in die Messkammer 7 hineingedrückt und verlässt diese durch die Einlassöffnung 9
(Ausblasphase 29). Während dieser Zeit tritt keine oder eine
viel kleinere zusätzliche Verbrennungswärme am Gassensor 11
auf. Der Gassensor 11 ist also während der Ausblasphase 29 etwas kälter als während der Einsaugphase 28 und die elektrische Leitfähigkeit σ steigt deshalb während der Ausblasphase 29 an. Der Gassensor 11 zeigt also eine Leitfähigkeit σ, die wieder aus einem DC- und einem AC-Anteil besteht, wobei der letztere eine Frequenz aufweist, die identisch ist mit der Schwingungsfrequenz des Luftbewegungsgenerators 4 und eine Amplitude hat, die von der Konzentration der reduzierenden Gase im zu untersuchenden Gasgemisch abhängt.

Durch die Auswerteschaltung 3 wird die elektrische Leitfähigkeit σ der Heizwicklung 10 in ein Spannungssignal umgewandelt, das ebenfalls aus einem DC-Signal und einem diesem überlagerten AC-Signal besteht. Die Auswerteschaltung 3 trennt diese beiden Signale und bestimmt die Amplitude des AC-Signals. Sobald die AC-Amplitude einen vorbestimmten Wert überschreitet, gibt die Auswerteschaltung 3 ein Alarmsignal ab.

Gegenüber dem herkömmlichen Pellistorprinzip, das einen Mess- und einen Referenzpellistor benötigt, weist dieser erfindungsgemässe Gasdetektor 100 nur einen einzigen Pellistor als Gassensor 11 auf. Da der Luftbewegungsgenerator 4 nur sehr wenig elektrische Leistung benötigt, weist der hier beschriebene Gasdetektor 100 einen ca. nur halb so grossen Leistungsverbrauch auf wie herkömmliche Gasdetektoren, die ein Pellistorpaar enthalten. Da die Amplitude des AC-Signals sehr genau gemessen werden kann und durch Langzeitschwankungen von Druck, Temperatur und Luftfeuchtigkeit kaum beeinflusst wird, zeigt der hier beschriebene Gasdetektor 100 im Gegensatz zum herkömmlichen Pellistorprinzip auch noch kleinste Konzentrationen von reduzierenden Gasen im zu untersuchenden Gasgemisch zuverlässig an.

0157247

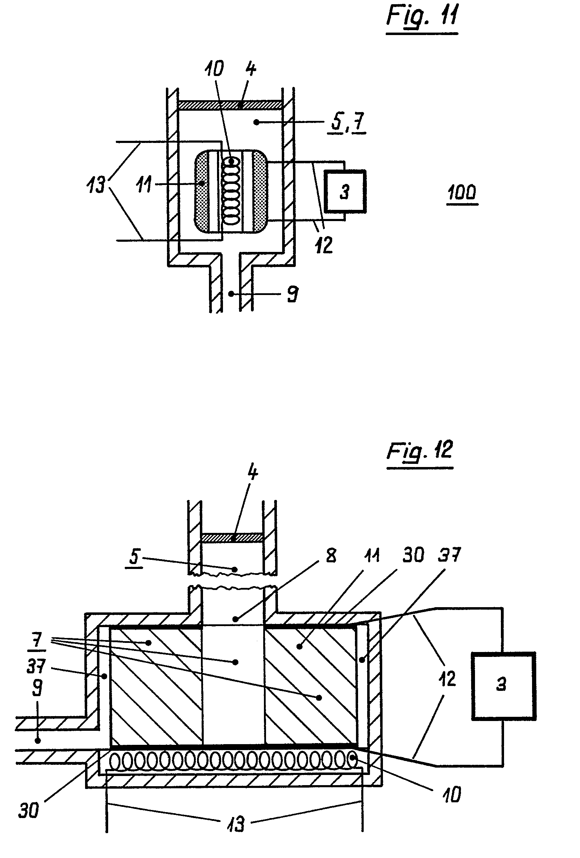

In Fig. 11 ist eine weitere Ausgestaltung eines im erfindungsgemässen Verfahren verwendeten Gasdetektors 100 dargestellt, der dank seiner äusserst einfachen Bauweise für die Massenproduktion sehr geeignet ist. Die Messkammer 7 und die Referenzkammer 5 gehen ohne eine Oeffnung 8 direkt ineinander über, der Gassensor 11 mit seiner Heizung 10 befindet sich also in einer Kammer, die zugleich Messkammer 7 und Referenzkammer 5 ist, wobei eine Wand dieser Kammer als Luftbewegungsgenerator 4 ausgebildet ist.

Das Verhältnis des Volumens der Messkammer = Referenzkammer zur Summe der Volumina von Gassensor 11 und Heizung 10 beträgt in diesem Beispiel bei der Verwendung eines TGS-812 Gassensors ca. 5 : 1. Da im erfindungsgemässen Gasdetektor 100 zum Nachweis von Gasen ein Wechselspannungssignal verwendet wird, das durch die Auswerteschaltung 3 proportional zum modulierten Anteil der elektrischen Leitfähigkeit des Gassensors 11 erzeugt wird, dessen Frequenz mit der Frequenz des Luftbewegungsgenerators 4 identisch ist und dessen Amplitude von Aenderungen der Temperatur, des Druckes und der relativen Luftfeuchte praktisch unabhängig ist, können mit einem solchen Gasdetektor 100 reduzierende Gase viel empfindlicher und störungsfreier gemessen werden, als mit einem der bisher üblichen Gassensoren, deren absolute elektrische Leitfähigkeit direkt zum Nachweis von Gasen verwendet wird.

In Fig. 12 ist eine Weiterentwicklung des in Fig. 11 beschriebenen Gasdetektors 100 gezeigt, wobei zur besseren Erkennbarkeit der Details die Messkammer 7 in einem etwa dreissigmal grösseren Massstab gezeichnet ist als die Referenzkammer 5. Der Gassensor 11 besteht aus einem hochporösen Stück eines quadratischen Metalloxidhalbleiters, mit den Massen von 1 x 1 x 1 $mm^3$, das in der Mitte ein Loch aufweist.

Der Gassensor 11 hat elektrische Ableitungen 12, die auf zwei Elektroden 30 befestigt sind, die an zwei Seiten des quadratischen Metalloxidhalbleiters angebracht sind und zur Messung der elektrischen Leitfähigkeit σ des Gassensors 11 dienen. Ueber die Zuleitungsdrähte 13 wird der Heizung 10 elektrische Energie zugeführt, die zum Aufheizen des Gassensors 11 benötigt wird. Die Heizung 10 braucht nicht nur auf einer Seite des Gassensors 11 angebracht zu sein. Sie kann auch auf mehreren Seiten des Gassensors 11 diesen gleichmässig auf eine vorbestimmte Temperatur erwärmen. Zur Verringerung der Wärmeableitung sind die Wände der Messkammer 7 und Referenzkammer 5 bevorzugt aus einem wärmeisolierenden, gasdichten Material hergestellt.

Der Gassensor 11 füllt mit Ausnahme eines schmalen Spalts 37 an der Peripherie die gesamte Messkammer 7 aus, die bei der Oeffnung 8 direkt in die Referenzkammer 5 übergeht, deren Volumen durch den Luftbewegungsgenerator 4 periodisch verändert wird. Beim Bewegen des Luftbewegungsgenerators 4 wird das zu untersuchende Gasgemisch periodisch durch die Einlassöffnung 9 und durch den Gassensor 11 gesaugt (Einsaugphase 28) und danach in umgekehrter Richtung wieder ausgestossen (Ausblasphase 29). Durch die in Fig. 12 gezeigte Anordnung wird eine optimale Umsetzung der im zu untersuchenden Gasgemisch enthaltenen reduzierenden Gase erzielt. Das Verhältnis der Summe der Volumina von Gassensor 11 und Heizung 10 zur Summe der Volumina von Messkammer 7 und Referenzkammer 5 ist praktisch 1 : 1.

Mit einem Gasdetektor 100 in dieser Ausführungsform können noch kleinste Spuren von reduzierenden Gasen mit grösster Genauigkeit gemessen werden bei einem Leistungsverbrauch von nur ca. 50 mW elektrischer Energie, was ca. 1/10 der Leistung ist, die bisher verwendete Gassensoren benötigen und
die ausserdem wesentlich weniger empfindlich sind.

Die Auswertung der AC- und DC-Signale durch die Auswerteschaltung 3 kann im Prinzip durch eine beliebige dem Fachmann
bekannte Wechselspannungs- und Gleichspannungsschaltung ausgeführt werden.

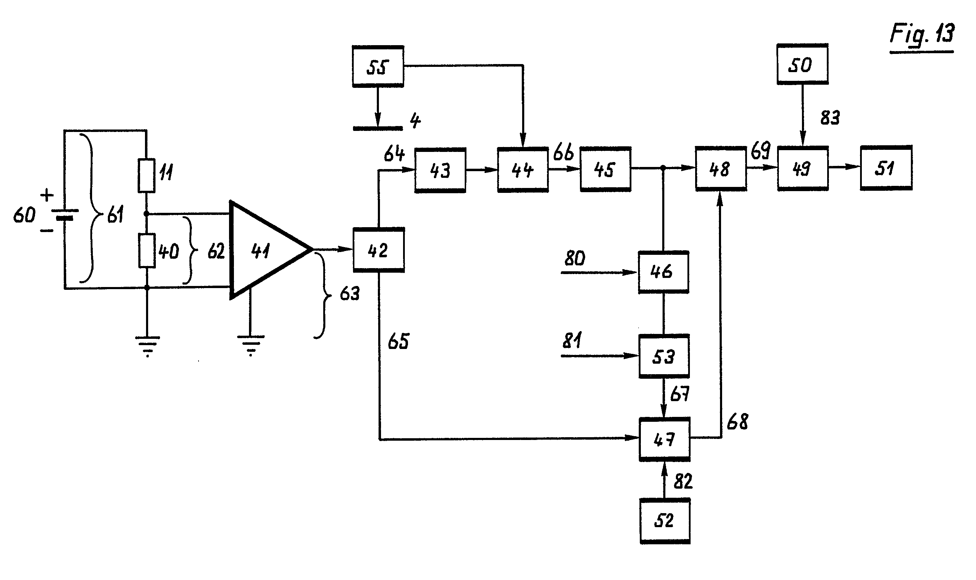

Fig. 13 zeigt ein geeignetes Beispiel in Blockform, das für
alle in den Fig. 6 bis 12 und 15 gezeigten Gasdetektoren 100
angewendet werden kann. Die Funktion der Schaltung in Fig.
13 ist anhand des CO-Nachweises in Abwesenheit oder Gegenwart
von $CH_4$ im folgenden beschrieben.

Der Gassensor 11 ist in Serie geschaltet mit einem Lastwiderstand 40, wobei über dieser Serieschaltung die Messspannung
61 der Spannungsquelle 60 liegt. Am Lastwiderstand 40 wird
die Spannung 62 vom Verstärker 41 abgegriffen und dank der
speziellen Verstärkerkennlinie in ein Spannungssignal 63 umgewandelt, das proportional zur elektrischen Leitfähigkeit σ
des Gassensors 11 ist. Der Verstärker 41 kann auch zweckmässigerweise eine logarithmische Charakteristik aufweisen, da
der erfindungsgemäss verwendete Gasdetektor 100 gemäss Gleichung (1) einen sehr grossen dynamischen Bereich für die auftretenden AC-Signale 64 hat. Das Spannungssignal 63, das aus
einem Gleichspannungsanteil (DC-Signal) mit überlagertem,
bei der Frequenz des Luftbewegungsgenerators 4 liegenden
Wechselspannungsanteil (AC-Signal) besteht, wird nun im AC-
DC-Entkoppler 42 in ein reines AC-Signal 64 (z.B. durch Auskoppeln über einen geeignet gewählten Kondensator) und in
ein reines DC-Signal mit dem DC-Wert 65 (z.B. durch einen
Integrator) aufgespalten. Das AC-Signal wird zweckmässigerweise in einem Bandpass 43 gefiltert und im (z.B. phasenempfindlichen) Gleichrichter 44 in ein der Amplitude des AC-Signals proportionales Gleichspannungssignal (AC-Amplitude 66) umgewandelt. Der Gleichrichter 44 wird mit der Frequenz des Treibers 55, der den Luftbewegungsgenerator 4 antreibt, gesteuert. Die AC-Amplitude 66 wird im Integrator 45 geglättet und erzeugt im Multiplikator 46 und Additionsverstärker 53 zusammen mit den Spannungen 80 und 81, die den Werten a bzw. b in Gleichung (2) entsprechen, einen fiktiven DC-Wert 67. Der Multiplikator 46, inklusive Additionsverstärker 53, die nach Gleichung (2) arbeiten

$$\text{fiktiver DC-Wert} = a \cdot \text{AC-Amplitude} + b \qquad (2)$$

benützen die beiden Konstanten a und b, die eine Gerade beschreiben, die der in Fig. 5 gestrichelten, ganz leicht gebogenen Linie für CO durch Regressionsrechnung optimal angepasst worden ist, wobei a die Steigung der Geraden und b deren Achsenabschnitt auf der Ordinate bedeuten. Diese Gerade liegt angenähert in der Mitte der Messwerte für CO für sehr feuchte und sehr trockene Atmosphäre. Der vom Multiplikator 46 inklusive Additionsverstärker 53 berechnete fiktive DC-Wert 67 liegt nun angenähert auf dieser in Fig. 5 eingezeichneten, gestrichelten Linie. Im Differenzverstärker 47 wird jetzt der fiktive DC-Wert 67 vom gemessenen DC-Wert 65 subtrahiert. Stimmen fiktiver und wahrer DC-Wert innerhalb der in Fig. 5 gezeigten, durch wechselnde relative Feuchten bedingten Grenzen für CO überein, so ist klar die Anwesenheit von nur CO als reduzierendes Gas im zu untersuchenden Gasgemisch bewiesen.

Der Differenzverstärker 47 bildet den negativen Absolutwert von (DC-Wert 65 - DC-Wert 67) und gibt unter zusätzlicher Addition der Spannung 82, die in Fig. 5 dem zur Ordinate parallelen Abstand k zwischen den Bändern für CO und $CH_4$ entspricht und laut Fig. 13 durch die Spannungsquelle 52 erzeugt wird, eine Spannung 68 ab, die dem Wert k-|(wahrer DC - fiktiver DC)| entspricht. Im Multiplikator 48 wird die geglättete AC-Amplitude 66 mit der Spannung 68 multipliziert. Stimmen fiktiver und wahrer DC-Wert überein, so erfolgt die Multiplikation im Multiplikator 48 mit dem Faktor k. Stimmen andererseits fiktiver 67 und wahrer 65 DC-Wert innerhalb der in Fig. 5 gezeigten Grenzen für CO nicht überein, so stammt die am Gassensor beobachtete Aenderung der elektrischen Leitfähigkeit $\sigma$ offensichtlich nicht nur vom gefährlichen CO, sondern auch noch vom $CH_4$. Der Differenzverstärker 47 erzeugt deshalb eine Spannung 68, die im Multiplikator 48 die geglättete AC-Amplitude 66 mit einem Wert zwischen 0 und k multipliziert. Die nach dem Multiplikator 48 vorliegende Spannung 69 entspricht nun einer AC-Amplitude, die nur noch von CO herrührt.

Der Komparator 49 vergleicht diese "Nur-CO-AC-Amplitude" 69 mit einer Referenzspannung 83 des Sollwertgebers 50 und steuert eine Alarmschaltung 51 an, sobald die "Nur-CO-AC-Amplitude" 69 einen vorgegebenen Schwellenwert überschreitet. Verlaufen die Bänder für die Gase CO und $CH_4$ in Fig. 5 nicht parallel, so wird die Konstante k abhängig von der AC-Amplitude und muss durch einen in Fig. 13 nicht gezeigten weiteren Multiplikatorverstärker entsprechend der AC-Amplitude korrigiert werden, was zweckmässig durch Beeinflussung der Spannungsquelle 52 erfolgt. Können die Bänder für CO und $CH_4$ in Fig. 5 nicht genügend genau durch Geraden approximiert werden, so werden die in Fig. 5 eingezeichneten gestrichelten Kurven durch ein allgemeines Polynom n-ten Grades dargestellt, was anstelle der zwei Spannungen 80 und 81 nun n+1 Spannungen benötigt und entsprechend mehr Additions- und Multiplikationsverstärker.

Die in Fig. 13 beschriebene Schaltung kann auch in der Weise verwendet werden, dass nur die Anwesenheit eines bestimmten reduzierenden Gases in Anwesenheit von anderen reduzierenden Gasen durch geeignete optische oder akustische Mittel angezeigt wird.

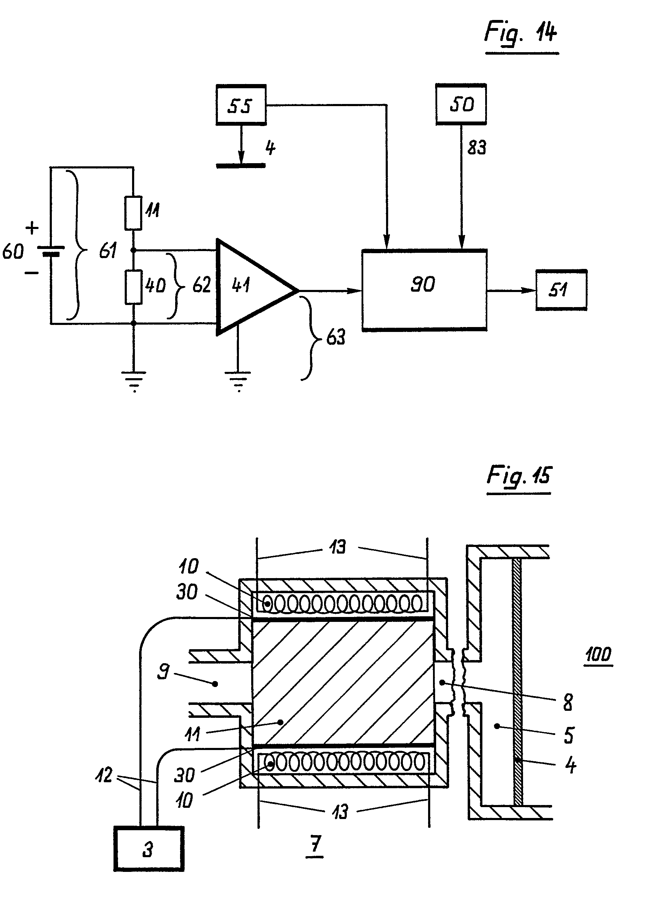

Selbstverständlich können die in Fig. 13 beschriebenen Funktionen nicht nur in herkömmlicher Weise durch Analogschaltungen bewirkt werden, sondern können mittels dem Fachmann bekannter Digitalschaltkreise unter Verwendung eines Mikroprozessors realisiert werden, siehe Fig. 14. Das vom Verstärker 41 aufbereitete Signal 63 wird in diesem Falle vom digitalen, einen Mikroprozessor enthaltenden, Schaltkreis 90 in bekannter Weise digitalisiert, wobei die Abtastfrequenz zweckmässigerweise mindestens 2 mal, besser aber 10 mal, insbesondere 100 mal, höher liegt als die Frequenz des Luftbewegungsgenerators 4, bzw. seines Treibers 55, die als Referenzfrequenz dem digitalen Schaltkreis 90 zur Verfügung gestellt wird. Im Vergleich zur Referenzspannung 83 des Sollwertgebers 50 berechnet und entscheidet der digitale Schaltkreis 90, ob ein bestimmtes reduzierendes Gas in gefährlicher Konzentration vorliegt und steuert gegebenenfalls eine Alarmschaltung 51 an.

Fig. 15 zeigt eine Weiterentwicklung des in Fig. 12 gezeigten erfindungsgemässen Gasdetektor 100, wobei zur besseren Erkennbarkeit der Details die Messkammer 7 in einem etwa dreissigmal grösseren Massstab gezeichnet ist als die Referenzkammer 5. Der Gassensor 11 mit den Massen $0,8 \times 0,8 \times 0,5$ $mm^3$ besteht aus einem Stück hochporösen Metalloxids, das auf zwei Seiten die Elektroden 30 trägt, von zwei Heizungen 10 auf eine vorbestimmte Temperatur gebracht wird und die Messkammer 7 völlig ausfüllt. Der Luftbewegungsgenerator 4, der eine Wand der Referenzkammer 5 bildet, besteht aus einer
Metallmembran von 30 mm Durchmesser, die von einem handelsüblichen Piezoschwinger angetrieben wird und dessen kleine
Bewegungsamplituden ausreichen, um das zu untersuchende Gasgemisch durch die Einlassöffnung 9, durch den Gassensor 11
und die Verbindungsöffnung 8 in die Referenzkammer 5 zu saugen und anschliessend in umgekehrter Richtung aus der Referenzkammer 5 durch die Verbindungsöffnung 8 und durch den
Gassensor 11 und die Einlassöffnung 9 wieder auszublasen.
Da die Wände der Messkammer 7 und der Referenzkammer 5 aus
einem gasdichten Material mit sehr kleiner Wärmeleitfähigkeit
bestehen, benötigt dieser Gasdetektor 100 nur ca. 80 mW elektrische Leistung und kann auch noch kleinste Spuren von reduzierenden Gasen äusserst genau nachweisen, indem die elektrische Leitfähigkeit $\sigma$ des Gassensors 11 über die Elektroden 30 und die Zuleitungen 12 durch die Auswerteschaltung
3 gemessen wird und in einen DC-Wert und eine AC-Amplitude
aufgeteilt wird, wobei die AC-Amplitude von einem Anteil der
elektrischen Leitfähigkeit $\sigma$ stammt, der mit der Frequenz
des Luftbewegungsgenerators 4 moduliert ist. Durch einen Vergleich des DC-Werts mit der AC-Amplitude kann die Auswerteschaltung 3 zusätzlich entscheiden, welches reduzierende Gas
detektiert worden ist. Soll der Gasdetektor 100 die Konzentration eines bestimmten reduzierenden Gases nachweisen, so
ist dies ebenfalls möglich, da die Auswerteschaltung 3, deren
Blockschaltbilder in Fig. 13 oder 14 gezeigt sind, die dazu
benötigten Umrechnungen selbständig durchführt. Diese Konzentrationsbestimmung ist unabhängig davon, ob noch andere reduzierende Gase ausser dem speziell zu detektierenden Gas im
zu untersuchenden Gasgemisch enthalten sind.

Das Verhältnis des Referenzkammervolumens zum Messkammervolumen beträgt 5:1, das Verhältnis der Summe der Volumina von Referenzkammer und Messkammer zur Summe der Volumina von Gassensor 11 und Heizung 10 beträgt ca. 6:1 für diesen experimentell erprobten Gasdetektor 100.

Figur 16 zeigt eine Weiterentwicklung des in Figur 12 dargestellten Gasdetektors 100. Der Gassensor 11 besteht dabei aus einem quaderförmigen Stück Metalloxid, wobei auf zwei gegenüberliegenden Seiten die Elektroden 32 aufgebracht sind. Bei dieser Ausgestaltung stellt das Material des Gassensors 11 die Messkammer 7 dar. Das poröse Metalloxid kann ein Material sein, wie es in der EP-A-120 203, EP-PA 84 109 653 oder EP-PA 84 107 849 beschrieben ist. Die Messkammer 7, d.h. der Metalloxidblock weist die Masse 2 x 2 x 1 mm auf, wobei in der dargestellten Ausgestaltung die Elektroden 32 Boden und Decke der Messkammer bilden während die Seitenwände des Metalloxidblocks die Oeffnung 9, d.h. die Verbindung mit der Aussenatmosphäre bilden. Dadurch wird ein ausserordentlich schneller Gasaustausch gewährleistet und der Strömungswiderstand des Gassensors 11 gering gehalten. Die Referenzkammer 5 ist unmittelbar auf eine der Elektroden 32 aufgesetzt, und die Verbindungsöffnung 8 befindet sich in dieser Elektrode 32. Der Luftbewegungsgenerator 4 am Ende der Referenzkammer 5 ist ein Minilautsprecher mit ca. 20 mm Durchmesser, und die Referenzkammer 5 ist ein Rohr aus einem Material mit geringer Wärmeleitfähigkeit.

Durch die Bewegungen des Luftbewegungsgenerators 4 wird das zu untersuchende Gasgemisch durch die Wände und das Material des Gassensors 11 (und damit durch die Messkammer 7) in die Referenzkammer 5 gesaugt und anschliessend in umgekehrter Richtung wieder ausgeblasen. Die Heizung 10 ist auf einer wärmeisolierenden Unterlage 38 befestigt; sie benötigt daher nur etwa 250 mW elektrische Heizleistung und kann auch noch kleinste Spuren von reduzierenden Gasen nachweisen. Die elektrische Auswertung der Signale erfolgt in der Auswerteschaltung wie für das vorstehende Beispiel beschrieben.

Diese Konstruktion bietet den Vorteil, dass das Volumen der Messkammer 7 durch das Volumen des Gassensors 11 exakt bestimmt ist. Dadurch wird eine hohe Reproduzierbarkeit der AC- und DC-Signale für verschiedene Gasdetektoren 100 erreicht.

0157247
C 274

P A T E N T A N S P R U E C H E

1. Verfahren zum Nachweis von reduzierenden Gasen in einem
zu untersuchenden Gasgemisch, insbesonere in Luft, mittels eines Gasdetektors (100), der eine gegen die Aussenluft abgeschlossene, einen Luftbewegungsgenerator (4)
aufweisende Referenzkammer (5) und eine mit der Referenzkammer (5) in Verbindung stehende, einen durch eine Heizung (10) auf bestimmte Temperaturen erhitzbaren Gassensor (11) aufweisende Messkammer (7) aufweist, und einer
Auswerteschaltung (3), wobei durch einen Luftbewegungsgenerator (4) abwechselnd das zu untersuchende Gasgemisch
und ein Referenzgas mit keinem oder einem geringen Gehalt
an reduzierenden Gasen aus der Referenzkammer (5) in die
Messkammer (7) gefördert wird, dadurch gekennzeichnet,
dass als Gassensor (11) ein Metalloxid-Halbleiter, dessen
elektrische Leitfähigkeit ($\sigma$) direkt von der Konzentration an reduzierenden Gasen abhängt, verwendet wird, und
dass die sich periodisch ändernde elektrische Leitfähigkeit ($\sigma$), die sich aus einem konstanten Anteil und einem
mit der Frequenz des Luftbewegungsgenerators (4) modulierten, sein Vorzeichen wechselnden Anteil zusammensetzt, in der Auswerteschaltung (3) in ein Gleichspannungssignal (DC-Signal) und ein bei der Frequenz des
Luftbewegungsgenerators (4) liegendes Wechselspannungssignal (AC-Signal) aufgeteilt wird und dass mindestens
die AC-Amplitude (66) des Wechselspannungssignals (AC-
Signal) zum Nachweis von reduzierenden Gasen ausgewertet
wird.

0157247

2.  Verfahren gemäss Patentanspruch 1, dadurch gekennzeichnet, dass die Frequenz der aus Einsaugphase (28) und Ausblasphase (29) bestehenden Gasaustauschperiode (27) zwischen 0,01 und 50 Hz, vorzugsweise zwischen 0,1 und 10 Hz, insbesondere etwa 2 Hz, beträgt.

3.  Verfahren gemäss einem der Patentansprüche 1 und 2, dadurch gekennzeichnet, dass der Gassensor (11) auf eine Temperatur im Bereich von 150 bis 450 °C, vorzugsweise etwa 350 °C, aufgeheizt wird.

4.  Verfahren gemäss einem der Patentansprüche 1 bis 3, dadurch gekennzeichnet, dass in der Auswerteschaltung (3) die Amplitude des AC-Signals und der Wert des DC-Signals ermittelt und daraus unter Zuhilfenahme von Kenngrössen des Gassensors (11) die Anwesenheit eines bestimmten reduzierenden Gases, vorzugsweise dessen genaue Konzentration in Gegenwart von anderen reduzierenden Gasen bestimmt wird.

5.  Verfahren gemäss einem der Patentansprüche 1 bis 4, dadurch gekennzeichnet, dass in der Auswerteschaltung (3) bei Ueberschreiten eines bestimmten Wertes der AC-Amplitude (66) ein Alarmsignal ausgelöst wird.

6.  Verfahren gemäss Patentanspruch 5, dadurch gekennzeichnet, dass in der Auswerteschaltung (3) das Verhältnis des Gleichspannungssignals (DC-Signal) zur Amplitude des Wechselspannungssignals (AC-Signal) gebildet wird und dass ein Alarmsignal nur ausgelöst wird, wenn dieses Verhältnis einen bestimmten Wert aufweist.

7.  Vorrichtung zur Durchführung des Verfahrens gemäss einem der Patentansprüche 1 bis 6, mit einem Gasdetektor (100), der eine gegen die Aussenluft abgeschlossene, einen Luftbewegungsgenerator (4) aufweisende Referenzkammer (5)
und eine mit der Referenzkammer (5) in Verbindung stehende, einen durch eine Heizung (10) auf bestimmte Temperaturen erhitzbaren Gassensor (11), der bei Einwirkung von
reduzierenden Gasen seinen elektrischen Widerstand ändert, aufweisende Messkammer (7) aufweist, und einer Auswerteschaltung (3), dadurch gekennzeichnet, dass der Gassensor (11) aus einem Halbleiter-Material, dessen elektrische Leitfähigkeit ($\sigma$) direkt von der Konzentration
an reduzierenden Gasen in dem zu untersuchenden Gasgemisch abhängt, vorzugsweise aus einem Metalloxid-Halbleiter-Material, besteht.

8. Vorrichtung gemäss Patentanspruch 7, dadurch gekennzeichnet, dass die Messkammer (7) eine Einlasssöffnung (9)
zum Einlass des zu untersuchenden Gasgemischs aufweist
und über mindestens eine Verbindungsöffnung (8) mit der
Referenzkammer (5) in Verbindung steht.

9. Vorrichtung gemäss einem der Patentansprüche 7 und 8,
dadurch gekennzeichnet, dass der Gasdetektor (100) als
Luftbewegungsgenerator (4) eine elektromagnetisch, elektrostatisch, piezoelektrisch oder thermomechanisch erregbare Membran in der Art eines Lautsprechers, eine Piezo-
Folie aus PVDF, ein bimorphes piezoelektrisches oder bimetallisches Element oder eine durch Mikrolithorgraphie
erzeugte dünne Siliziumfolie aufweist.

10. Vorrichtung gemäss einem der Patentansprüche 7 und 8,
dadurch gekennzeichnet, dass der Gasdetektor (100) als
Luftbewegungsgenerator (4) einen beweglichen Kolben enthält, der das zu untersuchende Gasgemisch während der
Einsaugphase (28) durch den mit einer Heizung (10) beheizten Gassensor (11) hindurch und teilweise über die-

0157247

sen hinweg in der Weise saugt und dann zurückdrückt,
dass praktisch das gesamte in der Messkammer (7) befindliche zu untersuchende Gasgemisch mit der Oberfläche des
Gassensors (11) in Berührung kommt, wodurch eine vollständige Umsetzung der reduzierenden Gase an der Oberfläche des Gassensors (11) erfolgt und während der Ausblasphase (29) der Messkammer (7) durch Zurückdrücken
des Kolbens ein praktisch von reduzierenden Gasen freies
zu untersuchendes Gasgemisch den Gassensor (11) passiert.

11. Vorrichtung gemäss einem der Patentansprüche 7 bis 10,
dadurch gekennzeichnet, dass das Volumen der Teile, die
das zu untersuchende Gasgemisch passiert, möglichst gering gehalten wird, so dass das Totvolumen auf ein Minimum reduziert wird.

12. Vorrichtung gemäss einem der Patentansprüche 7 bis 11,
dadurch gekennzeichnet, dass die Volumina von Messkammer
(7) und Referenzkammer (5) so aufeinander abgestimmt
sind, dass bei jeder Einsaugphase (28) frisches zu untersuchendes Gasgemisch durch die Einlassöffnung (9) in die
Messkammer (7) eingesaugt wird und bei jeder Ausblasphase
(29) mindestens ein Teil des zu untersuchenden Gasgemisches durch die Einlassöffnung (9) die Messkammer (7)
verlässt.

13. Vorrichtung gemäss einem der Patentansprüche 7 bis 12,
dadurch gekennzeichnet, dass das Verhältnis des Volumens
der Messkammer (7) zum Volumen der Referenzkammer (5)
des Gasdetektors (100) mehr als 1 : 100, vorzugsweise
mehr als 1 : 1000, beträgt.

14. Vorrichtung gemäss einem der Patentansprüche 7 bis 12,
dadurch gekennzeichnet, dass das Verhältnis des Volumens

0157247

der Referenzkammer (5) zum Volumen der Messkammer (7) mehr als 5 : 1 und das Verhältnis der Summe der Volumina von Referenzkammer (5) und Messkammer (7) zur Summe der Volumina von Gassensor (11) und Heizung (10) ca. 6 : 1 beträgt.

15. Vorrichtung gemäss Patentanspruch 7, dadurch gekennzeichnet, dass die Messkammer (7) aus einem zusammen mit der Heizung (10) auf einer wärmeisolierenden Unterlage angebrachten Block aus einem Halbleiter-Material, vorzugsweise einem Metalloxid-Halbleitermaterial besteht, auf dem Elektroden zur Messung des elektrischen Widerstandes direkt aufgebracht sind und an dessen einer Seite die Referenzkammer (5) angebracht ist.

16. Vorrichtung gemäss einem der Patentansprüche 7 bis 12 und 14, dadurch gekennzeichnet, dass Messkammer (7), Verbindungsöffnung (8) und Referenzkammer (5) des Gasdetektors (100) annähernd gleichen Querschnitt aufweisen.

17. Vorrichtung gemäss einem der Patentansprüche 7 bis 9 und 11 bis 16, dadurch gekennzeichnet, dass der Luftbewegungsgenerator (4) des Gasdetektors (100) eine schwingende Membran aufweist, und die Referenzkammer (5) so ausgebildet ist, dass sich die schwingende Membran des Luftbewegungsgenerators (4) während der Ausblasphase (29) dicht an eine vorzugsweise konusförmige Auflage anlegt.

18. Vorrichtung gemäss einem der Patentansprüche 7 bis 9 und 11 bis 17, dadurch gekennzeichnet, dass sich vor der schwingenden Membran des Luftbewegungsgenerators (4) des Gasdetektors (100) eine Schutzmembran befindet.

0157247

19. Vorrichtung gemäss einem der Patentansprüche 7 bis 19, dadurch gekennzeichnet, dass der Gasdetektor (100) in der Referenzkammer (5), vorzugsweise vor der Verbindungsöffnung (8), und/oder vor der Einlassöffnung (9) ein weiteres, durch Ausheizen in gewissen Zeitabständen regenerierbares Filter zur Adsorption von Gasen aufweist.

20. Vorrichtung gemäss einem der Patentansprüche 7 bis 19, dadurch gekennzeichnet, dass der Gasdetektor (100) in der Referenzkammer (5) einen weiteren Gassensor, vorzugsweise einen zur Bestimmung von Wasserdampf geeigneten Referenzsensor, aufweist.

21. Vorrichtung gemäss einem der Patentansprüche 7 bis 20, dadurch gekennzeichnet, dass die Einlassöffnung (9) des Gasdetektors (100) als Kapillare ausgebildet ist, deren Länge vorzugsweise mindestens 2 mm und deren Innendurchmesser vorzugsweise höchstens 1 mm, insbesondere etwa 0,3 mm beträgt.

22. Vorrichtung gemäss einem der Patentansprüche 7 bis 21, dadurch gekennzeichnet, dass die Wand des Gasdetektors (100) aus gasdichten, wärmeisolierendem Material besteht und dass das Material des Gassensors (11) der Wand der Messkammer (7) eng anliegt.

0157247

1/10

Fig.1

Fig.16

_Fig. 2a_

27      27

$\delta$
$[\Omega^{-1}]$

$t \ [s]$

28    29    28    29

_Fig. 2b_

Konzentration

$t \ [s]$

28    29    28    29

_Fig. 2c_

Fluss

Einsaugen

Ausblasen

$t \ [s]$

28    29    28    29

Fig. 3

Fig. 4

0157247

Fig. 5

*Fig.6*

_Fig. 7_

_Fig. 8_

0157247

_Fig. 9_

100

_Fig. 10_

100

_Fig. 11_

_100_

_Fig. 12_

Fig. 13

10 /10

_Fig. 14_

_Fig. 15_